# EUROPEAN PATENT APPLICATION

(11) **EP 4 223 365 A1**
(43) Date of publication of application: **09.08.2023**
(21) Application number: 22155600.4
(22) Date of filing: 08.02.2022
(51) Int. Cl.: A61N 5/06

(54) **A DEVICE FOR TRANSMITTING LIGHT WITHIN A BODY CAVITY**

(71) Applicant: Uvisa Health ApS, 2300 København S (DK)
(72) Inventor: Harris, Ella Jade Smoraczewska, 2300 København S (DK); Jones, Nicholas John, 2300 København S (DK); Simek, Gizem, 2300 København S (DK)
(74) Representative: Plougmann Vingtoft a/s

(57) **Abstract**

A device suitable for transmitting light from a device in a body cavity. The device includes a main tubular body having a distal end and a proximal end, at least one light source positioned within the main tubular body, an outer part, such as a plurality of tubular ribs and a switch. The device is configured to receive a battery to be electrically connected to the switch to power the light source, the battery and the switch preferably positioned within the main body , and wherein the device is adapted to be inserted within the body cavity, such as a vagina.

## Description

### FIELD OF THE INVENTION

The present invention relates to a device for transmitting light within a body cavity, such as a vagina, by using at least one light source positioned in the device.

### BACKGROUND OF THE INVENTION

Vaginitis is a condition commonly characterized by pain, itching, discomfort or unusual discharge most commonly caused by fungal and bacterial infections. Many women experience this condition recurrently throughout their. The condition can arise for a number of different reasons but is preferably a result of a change in the composition of the vaginal microbiome. This can happen due to, for example, hormonal changes, introduction of foreign pathogens, sensitivity to external compounds often found in personal care products such as soaps, detergents or tampons or introduction of semen.

A typical treatment is antibiotics for bacterial vaginosis, or antifungal medication for fungal infections, most often vulvovaginal candidiasis. Antibiotics are systemic and may cause side effects such as nausea, vomiting, diarrhea and headaches. Furthermore, antibiotics dominantly target bacteria, including both commensal and pathogenic species, leaving the vaginal microbiome susceptible to colonization by fungus species, leading to fungal infections. It is also documented that Gardnerella vaginalis, the bacteria most commonly associated with bacterial vaginosis has shown propensity for quickly developing antibiotic resistance when repeatedly treated with antibiotics. A similar tendency is observed in fungus species becoming resistant to antifungal treatments.

Hence, an alternative would be advantageous, and in particular a more efficient and/or reliable device for transmitting light within a body cavity, such as a vagina would be advantageous.

### OBJECT OF THE INVENTION

It is a further object of the present invention to provide an alternative to the prior art.

In particular, it may be seen as an object of the present invention to provide a device that solves the above mentioned problems of the prior art.

### SUMMARY OF THE INVENTION

Thus, the above described object and several other objects are intended to be obtained in a first aspect of the invention by providing a device for transmitting light within a body cavity, the device comprising:
a main tubular body having a distal end and a proximal end and further comprising two end elements, preferably circular shaped, one end element arranged at the distal end and the other end element arranged at the proximal end,
at least one light source positioned within the main tubular body, configured to provide light at a preselected wavelength and arranged to emit said light to the surroundings of the device,
an outer part, such as a plurality of tubular ribs, attached to the two end elements and configured to expand the body cavity by providing a bending of an outer surface of the outer part away from the main tubular body, such that a void between the main tubular body and the outer part is provided, preferably comprising air, such as air comprising oxygen,
a switch,

wherein the device is configured to receive a battery to be electrically connected to the switch to power the light source, the battery and the switch preferably positioned within the main body, and
wherein the device is adapted to be inserted within the body cavity, such as a vagina.

The invention is particularly, but not exclusively, advantageous for obtaining a comfortable and reusable device insertable within a body cavity, such as a vagina, for transmitting light to provide a light therapy to the surroundings inside the body cavity, while the device effectively expands the body cavity to increase the inner surface area of the body cavity affected by the light therapy.

In an embodiment of the device, at least one of the end elements may be moveable along a predetermined part of the tubular body and wherein the moveable end element may further comprises a locking element for locking the moveable end element at a position, wherein the device may have expanded the volume of the body cavity.

In an embodiment, the device may have increased the volume of the body cavity at least by 3%, such as at least 5%, such as at least 10%, such as at least 20%.

In an embodiment of the device, the bending of the outer surface may be provided by one end element being moveable and moved towards the other end element along the main tubular body, and may be locked by a locking element at a position, wherein the bending of the outer surface may have provided an expansion of the body cavity.

In an embodiment, the device may further comprising a groove and/or a through-going opening for providing air from the surroundings outside the body cavity to the void.

The invention is particularly, but not exclusively, advantageous for obtaining a device, which can provide air from the surroundings, such as the air available around the body cavity, through the groove and/or the through-going opening and into the void, thereby being able to provide oxygen preferably present in the air to the inside of the body cavity, which can limit the growth of harmful anaerobe microorganisms. Particularly, the increased oxygen concentration based on the air exchange through the groove and/or the through-going opening can change the body cavity conditions from being anaerobe to more aerobe.

In an embodiment, the device may further comprises a penetration cap, to guide the device into the body cavity.

In an embodiment, the device may further comprising a sensor to be electrically connected to the battery and configured to measure pH and/or temperature and/or colour of a tissue inside the body cavity and/or colour of a fluid inside the body cavity. In some embodiments, the device further comprising a plurality of sensors to be electrically connected to the battery, each sensor configured to measure pH and/or temperature and/or colour of a tissue inside the body cavity and/or colour of a fluid inside the body cavity.

In an embodiment of the device, the sensor may be arranged in the penetration cap and/or in the outer part.

In an embodiment, the device may further comprises an extending element, which extends outside the body cavity when the device may be inserted within the body cavity.

In an embodiment of the device, the extending element may be configured to facilitate signal transmission from the sensor to a receiving device positioned outside the body cavity during use of the device.

In an embodiment of the device, the outer part may be made of an expandable material, such as rubber, such as latex and/or the outer surface may be made of an expandable material, such as rubber, such as latex.

In an embodiment of the device, the exterior part may at least partly covers an outer surface of the body cavity, being the outer surface, which at least forms the entrance to the body cavity.

In an embodiment of the device, the exterior part may comprising a light source configured to provide light at a predetermined wavelength to the surroundings of the exterior part and electrically connected to the switch.

In an embodiment of the device, the light provided by the light source may be at a wavelength of at least 250 nm, such as at least 300 nm, such as at least 350 nm, such as at least 400 nm, but not at a wavelength above 500 nm.

The invention is particularly, but not exclusively, advantageous for obtaining a device for transmitting light, being light that is antimicrobial, which effectively can be used in a body cavity to decrease the amount of microorganisms present.

Particularly, the use of light within the range of 250-500 nm is known to be antimicrobial and/ antifungal and/or bactericidal.

In an embodiment of the device, the outer part may be a balloon structure and configured to be inflated such as to expand the body cavity.

In an embodiment, the device may further comprising a controller for controlling light intensity of the light source.

In an embodiment of the device, the length of the device may be between 3 cm and 15 cm, such as 5 cm and 11 cm, and/or the circumference of the device in a not expanded configuration may be between 4 cm and 12 cm, such as 6 cm and 10 cm.

In an embodiment of the device, the extending element may further comprises a communication element, and wherein the communication element may comprises a charger port for charging a battery, such as a rechargeable battery, and/or a transmitter for transmitting sensor data from the sensor.

In an embodiment, the device may further comprises a communication element arranged in the proximal end of the main tubular body, and wherein the communication element comprises a charger port for charging a battery, such as a rechargeable battery, and/or a transmitter for transmitting sensor data from the sensor.

In an embodiment, the device may further comprising a rechargeable battery arranged in the main tubular body and/or in the end elements and/or in the communication element, and configured to be recharged by a charger through a charger port.

In an embodiment, the device may further comprising a plurality of light sources, such as a plurality of LEDs, such as a plurality of light bulbs, and wherein each light source may have a preselected wavelength.

The invention is particularly, but not exclusively, advantageous for obtaining a device with a plurality of light sources wherein each light source has an individual wavelength, so that the device can be used to transmit light in a body cavity using a plurality of light wavelengths to decrease unwanted pathogens and/or microorganisms using preferably both UV and non-UV light.

In an embodiment, the body cavity may be a vagina.

In a second aspect, the present invention relates to a method for transmitting light within a body cavity, the method comprising:
- providing a device for transmitting light, the device comprising
   - a main tubular body having a distal end and a proximal end,
   - at least one light source positioned within the main tubular body, configured to provide light at a preselected wavelength and arranged to emit said light to the surroundings of the device,
   - a switch,
      wherein the device is configured to receive a battery to be electrically connected to the switch to power the light source, and wherein the device is adapted to be inserted within the body cavity, such as a vagina,
- inserting said device within a body cavity,
- activating using a switch at least one light source positioned within a main tubular body of the device, the at least one light source being configured to provide light at a preselected wavelength, and wherein said light source is arranged to emit said light to the surroundings of the device,
- removing said device from said body cavity.

In an embodiment, the method may firther comprises, upon inserting said device within a body cavity, expanding the body cavity by providing a bending of an outer part of the device, and preferably locking the bended outer part by a locking element, such as a cam locking mechanism.

This aspect of the invention is particularly, but not exclusively, advantageous in that the method according to the present invention may be implemented by providing a device according to the first aspect, which may significantly improve light transmitting in a body cavity, as a larger inner surface area may be available for light reception, when the body cavity is expanded due to the outer part of the device. This aspect of the invention is further, but not exclusively, advantageous in that the method may be implemented by providing a device that transmits light within a body cavity, and provides a therapy, such as a treatment, of the body cavity when inserted, which can inhibit the growth and decrease the amount of pathogens and other unwanted microorganisms present in the body cavity.

In an embodiment, the method may further comprises, upon activating said at least one light source, measuring pH and/or temperature and/or colour of a tissue inside the body cavity and/or colour of a fluid inside the body cavity, and preferably sending said measurements to a receiving device outside the body cavity or storing said measurements in a data storage comprised in the device.

In one embodiment of the method, the body cavity may be a vagina.

In one embodiment of the method, the device provided is a device according to aspect one.

The first and second aspect of the present invention may each be combined with any of the other aspects. These and other aspects of the invention will be apparent from and elucidated with reference to the embodiments described hereinafter.

### BRIEF DESCRIPTION OF THE FIGURES

The device according to the invention will now be described in more detail with regard to the accompanying figures. The figures show one way of implementing the present invention and is not to be construed as being limiting to other possible embodiments falling within the scope of the attached claim set.
Figure 1 illustrates a device for transmitting light with an extending element according to the present invention,
Figure 2 illustrates a device for transmitting light in a not expanded configuration according to the present invention,
Figure 3 illustrates a device for transmitting light in an expanded configuration according to the present invention,
Figure 4 illustrates in a half view one end element of a device for transmitting light according to the present invention,
Figure 5 illustrates in a partial view one end element of a device for transmitting light according to the present invention,
Figure 6 illustrates a locking mechanism according to the present invention.
Figure 7 illustrates a flow diagram of a method for providing light within a body cavity according to the present invention.

### DETAILED DESCRIPTION OF AN EMBODIMENT

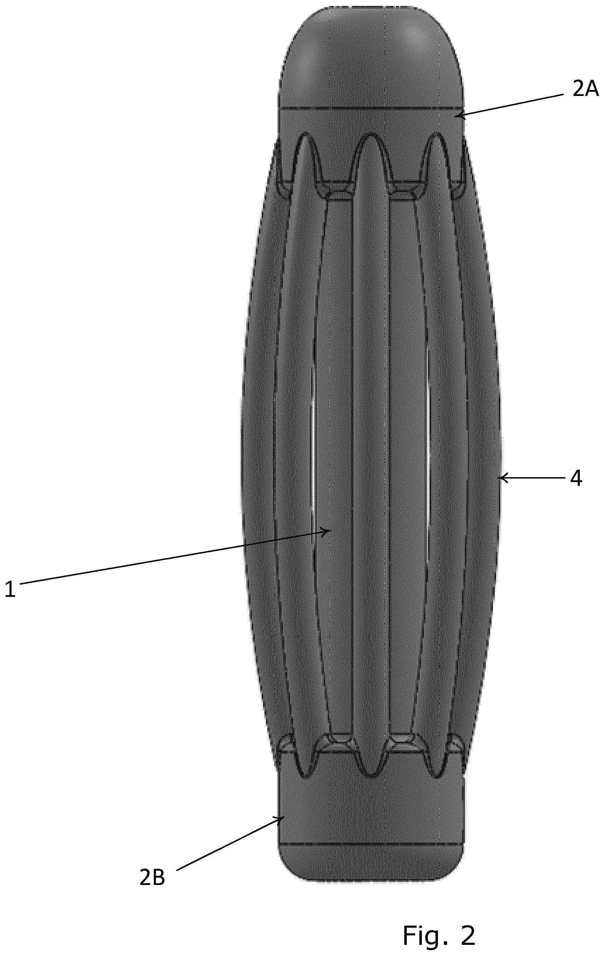

With reference to fig. 2, one embodiment of the invention will now be disclosed in greater details. Figure 2 illustrates a device for transmitting light within a body cavity, where the device comprises a main tubular body 1 having a distal end and a proximal end. The tubular body is typically transparent or translucent, but may also be opaque. In some embodiments, the tubular body may even be partly transparent and/or partly translucent and/or partly opaque.

The distal end of the tubular body 1 is characterised as the end, which is most distant compared to the proximal end to the entry of a body cavity, when the device is inserted in the body cavity. Contrary, is the proximal end closest compared to the distal end to the entry of a body cavity, when the device is inserted in the body cavity. The tubular body 1 further comprises two end elements 2A, 2B, preferably circular shaped but the end elements 2A, 2B may also have an outer shape being different, such as squared, such as rugged, such as rectangular or such as coned. One end element 2A is arranged at the distal end of the tubular body 1 and the other end element 2B is arranged at the proximal end of the tubular body 1. Preferably, the proximal end of the tubular body and/or the end element 2B arranged at the proximal end, is not within the body cavity when the device is inserted herein.

The device further comprises at least one light source positioned within the main tubular body 1, not shown in fig. 2, which is configured to provide light at a preselected wavelength and arranged to emit said light to the surroundings of the device. An outer part 3, such as a plurality of tubular ribs as shown in fig. 2, is typically attached to the two end elements 2A, 2B and configured to expand the body cavity by providing a bending of an outer surface 4 of the outer part 3 away from the main tubular body 1, such that a void 10 between the main tubular body 1 and the outer part 3 is provided. The outer surface 4 of the outer part 3 refers to the surface of the outer part, such as the surface of the ribs.

The outer part 3 may be pre-bended as shown in fig. 2. In order to power the light source(s) the device is configured to receive a battery to be electrically connected to a switch.

It is noted that "expand the body cavity" preferably refers to an increase in a volume or in the three-dimensional-space of the body cavity.

The device is typically used on an on/off basis, and adapted to be inserted within the body cavity, such as a vagina. Based on the shape of the body cavity, wherein the device is to be inserted, the overall shape of the device may be altered to fit the shape of the body cavity. The device is preferably retained in the body cavity for a predetermined amount of time, and then turned off by using a switch before removed. In some embodiments, the device may be turned off outside the body cavity.

In preferred embodiments, the length of the device is between 3 and 15 cm, such as 5 and 11 cm, and the circumference of the device in a not expanded configuration is between 4 and 12 cm, such as 6 and 10 cm.

It is noted that "a not expanded configuration" preferably refers to the device being in a configuration as illustrated in fig. 2, where the outer part 3 shown as ribs are limited to a pre-bended, not-bended or in a neutral position, which is preferably the position of ribs when the device is not inserted into a body cavity.

In some embodiments, the void comprises air, typically air comprising oxygen.

The device preferably comprises a switch located at a predetermined location on the device, electrically connected to an energy source, such as a battery, and the light source, and wherein the switch is configured to turn on/off the light source.

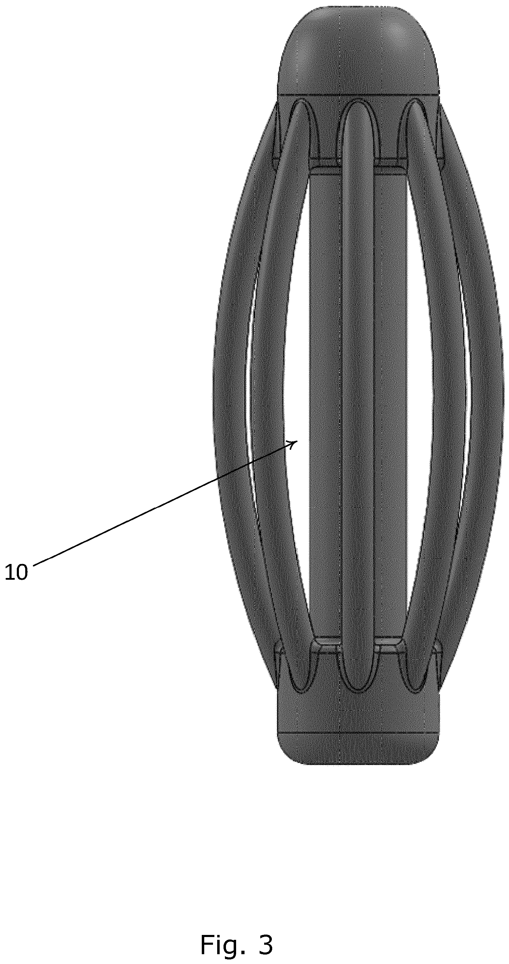

With reference to figure 3, the device may also be in an expanded configuration. It is noted that "an expanded configuration" preferably refers to the outer part 3 of the device, shown as ribs in figure 3, being bended to such a degree that the device expand the body cavity when inserted.

In a preferred embodiment, the expanded configuration is achieved by at least one of the end elements 2A, 2B being moveable along a predetermined part of the tubular body 1. The moveable end element 2A, 2B preferably comprises a locking element 8 for locking the moveable end element 2A, 2B at a position, wherein the device has expanded the volume of the body cavity.

The locking element 8 is preferably arranged along and/or within the main tubular 1 at a position preferably being in the proximal end of the main tubular body 1.

When the device is in an expanded configuration, the void 10 is preferably larger and thereby preferably comprises more air, such as air with oxygen, than when the device is in a not expanded configuration.

In some embodiments, the device has increased the volume of the body cavity at least by 3%, such as at least 5%, such as at least 10%, such as at least 20%. The increase of volume can be calculated based on a point of reference, which is a volume of the body cavity before the device is inserted therein. Alternatively, an internal diameter of the body cavity is used instead of volume, and wherein an increase in diameter based on a point of reference reflects the amount the device has increased the body cavity, such as at least by 3%, such as at least 5%, such as at least 10%, such as at least 20%.

It is noted that the "the internal diameter" preferably refers to an internal diameter measured from one wall of the body cavity to an opposite wall of the body cavity, and typically a point of reference is chosen based on the largest internal diameter before the device is inserted.

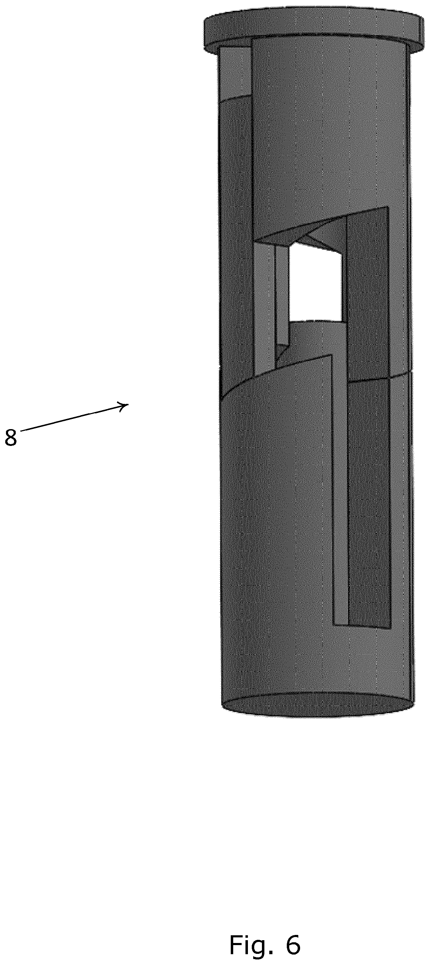

In another embodiment, the bending of the outer surface 4 is provided by one end element 2A, 2B being moveable and moved towards the other end element 2A, 2B along the main tubular body 1, and locked by a locking element 8 at a position, wherein the bending of the outer surface has provided an expansion of the body cavity, which preferably increases the inner surface area of the body cavity. One such locking element 8 is illustrated in fig. 6 in the form of a cam locking mechanism.

It is noted that the "inner surface area" preferably refers to a surface area on the inside of the body cavity, being the surface area within the body cavity.

In other embodiments, the outer part 3 is a balloon structure and configured to be inflated, so as to expand the body cavity.

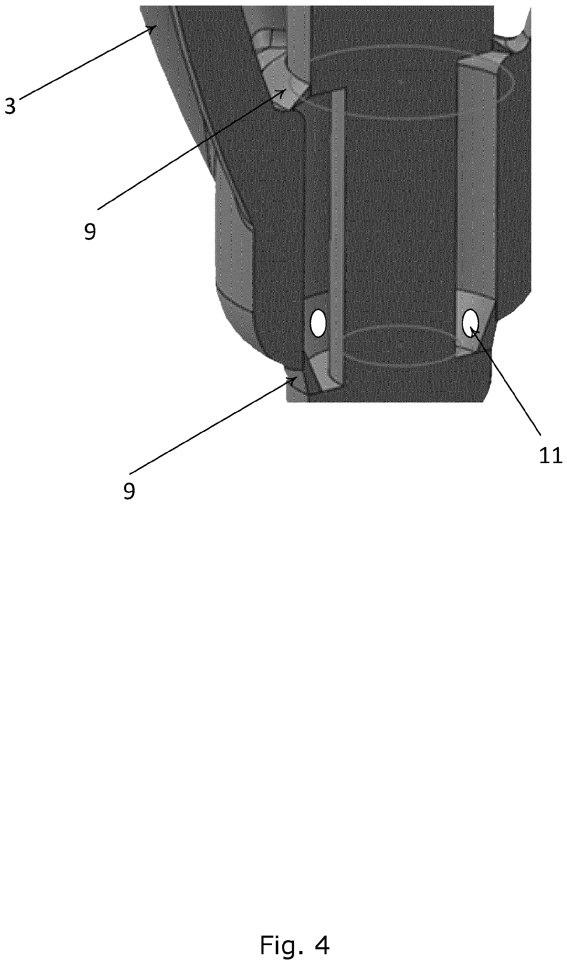

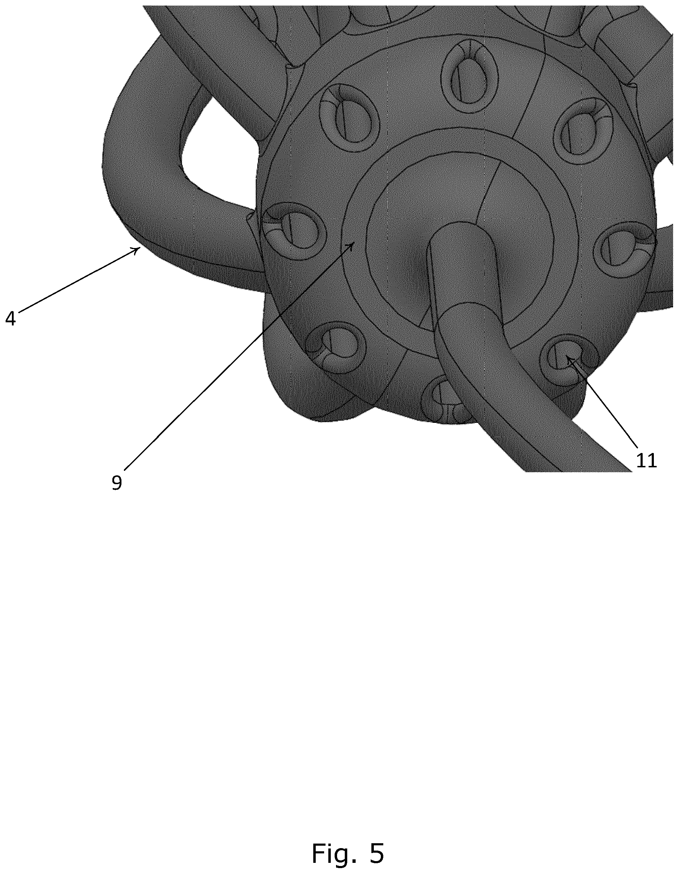

With reference to figure 4, which is a cross-sectional view of one of the end elements 2A, 2B, one embodiment of the invention is illustrated, where air from the surroundings outside the body cavity can be provided to the void 10 through through-going openings 11 in couplers 9 arranged between the end element 2A, 2B and the main tubular body 1. In the figure, through-going openings 11 are only illustrated in one of the couplers 9, but similar through-going openings 11 is preferably in the coupler being closest to the outer part 3. Turning to fig. 5, another embodiment with respect to the position of the through-going openings 11 is illustrated, wherein the through-going openings 11 are positioned within the end element 2A, 2B, for providing air from the surroundings outside the body cavity to the void of the device. The through-going openings 11 can be positioned in both end elements 2A, 2B or in one of them. Preferably, the through-going openings 11 are located in the proximal end of the tubular body, being the end element 2B.

In another embodiment, the device further comprising a groove for providing air from the surroundings outside the body cavity to the void 10.

The environment inside a body cavity is typically anaerobic, meaning that free oxygen is absent, which could favours bacteria and other microorganisms being obligate anaerobes and aerotolerant anaerobes. By providing air from the surroundings outside the body cavity, which preferably will be air comprising oxygen, the environment inside the body cavity may change from being anaerobic to contain some free oxygen and thereby be more aerobic. By providing either a groove and/or a plurality of grooves and/or a through-going opening 11 and/or a plurality of through-going openings 11 in the device, the effect obtained by providing a more aerobic environment inside a body cavity can be obtained.

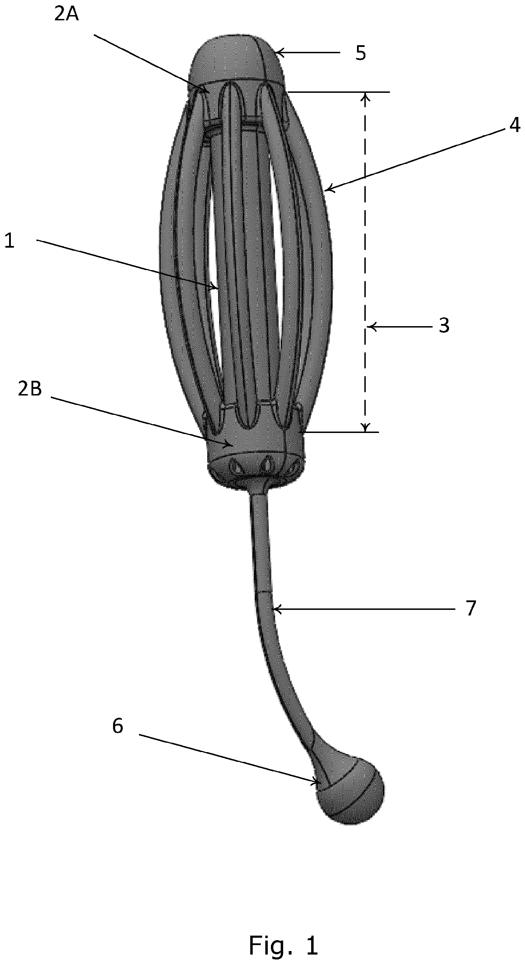

In a further embodiment, the device further comprises a penetration cap 5, to guide the device into a body cavity. In fig. 1 an example of such a penetration cap 5 is illustrated.

In a further embodiment, the device further comprising a sensor to be electrically connected to the battery and configured to measure pH and/or temperature and/or colour of a tissue and/or colour of a fluid inside the body cavity. The fluid is typically but not limited to bodily mucous.

Measuring pH, temperature, colour of a tissue or the colour of a fluid inside the body cavity using a sensor arranged within the device may be used to provide information regarding a current health status. A health status may be used to determine the amount of time the device should be inserted and retained in the body cavity, such as a vagina.

Preferably, the sensor is arranged in the penetration cap 5 and/or in the outer part.

In a further embodiment, the device further comprises an extending element 7, which extends outside the body cavity when the device is inserted within the body cavity. Such as by easing a retrieval of the device from the body cavity. Preferably flexible.

Typically, the extending element 7 is configured but not limited to facilitate signal transmission from the sensor to a receiving device positioned outside the body cavity during use of the device.

In some embodiments, the extending element 7 further comprises a communication element 6, and wherein the communication element 6 comprises a charger port for charging a battery, such as a rechargeable battery, and/or a transmitter for transmitting sensor data from the sensor. Alternatively, the communication element 6 may be arranged in one end of the main tubular body 1.

In a further embodiment, the outer part 3 is made of an expandable material, such as rubber, such as latex and/or the outer surface 4 is made of an expandable material, such as rubber, such as latex. The expandable material is preferably transparent or translucent, to allowing light to pass through the material.

In a further embodiment, the device further comprising an exterior part, wherein the exterior part at least partly covers an outer surface of the body cavity, being the surface, which at least forms the entrance to the body cavity. The area forming an entrance to a body cavity is not defined, but may preferably be predetermined for each application. For example, the surface which at least forms the entrance around a vagina can be predetermined to be at least the area of a circle around the entrance having at least a diameter of 1 cm, such as at least 2cm, such as at least 2.5 cm, but not exceeding 7.5 cm.

Preferably, the exterior part comprises a light source configured to provide light at a predetermined wavelength to the surroundings of the exterior part and electrically connected to the switch.

In a further embodiment, the light provided by the light source is at a wavelength of at least 250 nm, such as at least 300 nm, such as at least 350 nm, such as at least 400 nm, but not at a wavelength above 500 nm.

Both UV light below 400 nm and blue light above 400 nm until 500 nm is known to have antimicrobial and/or germicidal and/or bactericidal and/or antifungal properties and can be utilized to decrease pathogens and other unwanted microorganisms in a body cavity, while preferably protecting beneficial microorganisms by such as controlling the light intensity, the duration of light transmitting from the device to the body cavity. In some embodiments, the device comprising a plurality of light sources, such as a plurality of LEDs, such as a plurality of light bulbs, and wherein each light source has a preselected wavelength.

In a further embodiment, the device further comprising a controller for controlling light intensity of the light source. A switch may also be arranged in the controller, and configured to turn on/off the light source.

In a further embodiment, the device further comprising multiple light sources, such as multiple LEDs, such as multiple light bulbs, and wherein each light source has a preselected wavelength.

In a further embodiment, the device comprising a rechargeable battery arranged in the main tubular body 1 and/or in any of the end elements 2A, 2B and/or in the communication element 6, and configured to be recharged by a charger in a charger port.

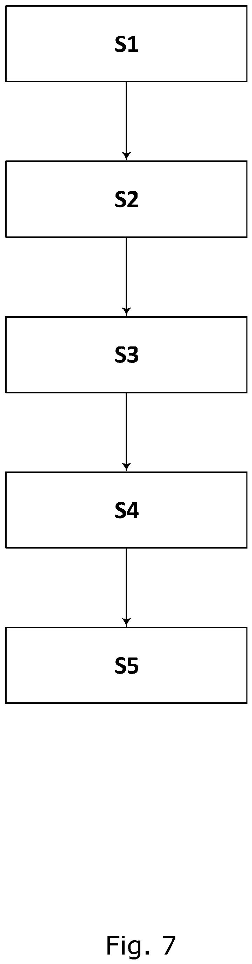

Figure 7 schematically illustrates through five steps a preferred embodiment of a method for transmitting light within a body cavity, the method comprising:
**51** providing a device for transmitting light within a body cavity,
**S2** inserting said device within a body cavity,
**S3** expanding the body cavity by providing a bending of an outer part of the device,
**S4** activating using a switch at least one light source positioned within a main tubular body of the device, the at least one light source being configured to provide light at a preselected wavelength, and wherein said light source is arranged to emit said light to the surroundings of the device,
**S5** removing said device from said body cavity.

In an embodiment of the method, upon expanding the body, the method further comprising the step of locking the bended outer part by a locking element, such as a cam locking mechanism.

In an another embodiment of the method, upon activating said at least one light source, the method further comprising the step of measuring pH and/or temperature and/or colour of a tissue inside the body cavity and/or colour of a fluid inside the body cavity, and preferably sending said measurements to a receiving device outside the body cavity or storing said measurements in a data storage comprised in the device.

To sum up, the invention relates to a device suitable for transmitting light from a device in a body cavity. The device includes a main tubular body having a distal end and a proximal end, at least one light source positioned within the main tubular body, an outer part, such as a plurality of tubular ribs and a switch. The device is configured to receive a battery to be electrically connected to the switch to power the light source, the battery and the switch preferably positioned within the main body , and wherein the device is adapted to be inserted within the body cavity, such as a vagina.

Although the present invention has been described in connection with the specified embodiments, it should not be construed as being in any way limited to the presented examples. The scope of the present invention is set out by the accompanying claim set. In the context of the claims, the terms "comprising" or "comprises" do not exclude other possible elements or steps. Also, the mentioning of references such as "a" or "an" etc. should not be construed as excluding a plurality. The use of reference signs in the claims with respect to elements indicated in the figures shall also not be construed as limiting the scope of the invention. Furthermore, individual features mentioned in different claims, may possibly be advantageously combined, and the mentioning of these features in different claims does not exclude that a combination of features is not possible and advantageous.

### ITEMIZED LIST

Item 1. A device for transmitting light within a body cavity, the device comprising:
   a main tubular body (1) having a distal end and a proximal end and further comprising two end elements (2A, 2B), preferably circular shaped, one end element (2A) arranged at the distal end and the other end element (2B) arranged at the proximal end,
   at least one light source positioned within the main tubular body, configured to provide light at a preselected wavelength and arranged to emit said light to the surroundings of the device,
   an outer part (3), such as a plurality of tubular ribs, attached to the two end elements (2A, 2B) and configured to expand the body cavity by providing a bending of an outer surface (4) of the outer part (3) away from the main tubular body (1), such that a void (10) between the main tubular body (1) and the outer part (3) is provided, preferably comprising air, such as air comprising oxygen,
   a switch,

   wherein the device is configured to receive a battery to be electrically connected to the switch to power the light source, the battery and the switch preferably positioned within the main body (1), and
   wherein the device is adapted to be inserted within the body cavity, such as a vagina.
Item 2. A device for transmitting light within a body cavity according to item 1, wherein at least one of the end elements (2A, 2B) is moveable along a predetermined part of the tubular body (1) and wherein the moveable end element (2A, 2B) further comprises a locking element (8) for locking the moveable end element (2A, 2B) at a position, wherein the device has expanded the volume of the body cavity.
Item 3. A device for transmitting light within a body cavity according to any of items 1 or 2, wherein the device has increased the volume of the body cavity at least by 3%, such as at least 5%, such as at least 10%, such as at least 20%. Item 4. A device for transmitting light within a body cavity according to any of the preceding items, wherein the bending of the outer surface (4) is provided by one end element (2A, 2B) being moveable and moved towards the other end element (2A, 2B) along the main tubular body (1), and locked by a locking element (8) at a position, wherein the bending of the outer surface has provided an expansion of the body cavity.
Item 5. A device for transmitting light within a body cavity according to any of the preceding items, further comprising a groove and/or a through-going opening (11) for providing air from the surroundings outside the body cavity to the void (10).
Item 6. A device for transmitting light within a body cavity according to any of the preceding items, wherein the device further comprises a penetration cap (5), to guide the device into a body cavity.
Item 7. A device for transmitting light within a body cavity according to any of the preceding items, further comprising a sensor to be electrically connected to the battery and configured to measure pH and/or temperature and/or colour of a tissue inside the body cavity and/or colour of a fluid inside the body cavity, and wherein the sensor is arranged in the penetration cap (5) and/or in the outer part (3).
Item 8. A device for transmitting light within a body cavity according to any of the preceding items, wherein the device further comprises an extending element (7), which extends outside the body cavity when the device is inserted within the body cavity.
Items 9. A device for transmitting light within a body cavity according to item 8 when dependant on item 7, wherein the extending element (7) is configured to facilitate signal transmission from the sensor to a receiving device positioned outside the body cavity during use of the device.
Item 10. A device for transmitting light within a body cavity according to any of the preceding items, wherein the outer part (3) is made of an expandable material, such as rubber, such as latex and/or the outer surface (4) is made of an expandable material, such as rubber, such as latex.
Item 11. A device for transmitting light within a body cavity according to any of the preceding items, further comprising an exterior part, wherein the exterior part at least partly covers an outer surface of the body cavity, being the outer surface, which at least forms the entrance to the body cavity.
Item 12. A device for transmitting light within a body cavity according to item 11, wherein the exterior part comprises a light source configured to provide light at a predetermined wavelength to the surroundings of the exterior part and electrically connected to the switch.
Item 13. A device for transmitting light within a body cavity according to any of the preceding items, wherein the light provided by the light source is at a wavelength of at least 250 nm, such as at least 300 nm, such as at least 350 nm, such as at least 400 nm, but not at a wavelength above 500 nm.
Item 14. A device for transmitting light within a body cavity according to any of the preceding items, wherein the outer part (3) is a balloon structure and configured to be inflated such as to expand the body cavity.
Item 15. A device for transmitting light within a body cavity according to any of the preceding items, further comprising a controller for controlling light intensity of the light source.
Item 16. A device for transmitting light within a body cavity according to any of the preceding items, wherein the length of the device is between 3 and 15 cm, such as 5 and 11 cm, and the circumference of the device in a not expanded configuration is between 4 and 12 cm, such as 6 and 10 cm.
Item 17. A device for transmitting light within a body cavity according to any of the preceding items when dependant on item 8, wherein extending element (7) further comprises a communication element (6), and wherein the communication element (6) comprises a charger port for charging a battery, such as a rechargeable battery, and/or a transmitter for transmitting sensor data from the sensor.
Item 18. A device for transmitting light within a body cavity according to any of the preceding items, further comprising a rechargeable battery arranged in the main tubular body (1) and/or in the end elements (2A, 2B) and/or in the communication element (6), and configured to be recharged by a charger through a charger port.
Item 19. A device for transmitting light within a body cavity according to any of the preceding items, further comprising a plurality of light sources, such as a plurality of LEDs, such as a plurality of light bulbs, and wherein each light source has a preselected wavelength.
Item 20. A method for transmitting light within a body cavity, the method comprising:
   - providing a device for transmitting light, the device comprising
      - a main tubular body having a distal end and a proximal end,
      - at least one light source positioned within the main tubular body, configured to provide light at a preselected wavelength and arranged to emit said light to the surroundings of the device,
      - a switch,
         wherein the device is configured to receive a battery to be electrically connected to the switch to power the light source, and wherein the device is adapted to be inserted within the body cavity, such as a vagina,
   - inserting said device within a body cavity,
   - activating using a switch at least one light source positioned within a main tubular body of the device, the at least one light source being configured to provide light at a preselected wavelength, and wherein said light source is arranged to emit said light to the surroundings of the device,
   - removing said device from said body cavity.
21. A method for transmitting light within a body cavity according to item 20, wherein, upon inserting said device within a body cavity, expanding the body cavity by providing a bending of an outer part of the device, and preferably locking the bended outer part by a locking element, such as a cam locking mechanism.
Item 22. A method for transmitting light within a body cavity according to any of items 20 or 21, wherein, upon activating said at least one light source, measuring pH and/or temperature and/or colour of a tissue inside the body cavity and/or colour of a fluid inside the body cavity, and preferably sending said measurements to a receiving device outside the body cavity or storing said measurements in a data storage comprised in the device.
Item 23. A method for transmitting light within a body cavity according to any of items 20-22, wherein, the device prodivided is a device according to any of items 1-19.

## Claims

1. A device for transmitting light within a body cavity, the device comprising:
a main tubular body (1) having a distal end and a proximal end and further comprising two end elements (2A, 2B), preferably circular shaped, one end element (2A) arranged at the distal end and the other end element (2B) arranged at the proximal end,
at least one light source positioned within the main tubular body, configured to provide light at a preselected wavelength and arranged to emit said light to the surroundings of the device,
an outer part (3), such as a plurality of tubular ribs, attached to the two end elements (2A, 2B) and configured to expand the body cavity by providing a bending of an outer surface (4) of the outer part (3) away from the main tubular body (1), such that a void (10) between the main tubular body (1) and the outer part (3) is provided, preferably comprising air, such as air comprising oxygen,
a switch,
wherein the device is configured to receive a battery to be electrically connected to the switch to power the light source, the battery and the switch preferably positioned within the main body (1), and
wherein the device is adapted to be inserted within the body cavity, such as a vagina.

2. A device for transmitting light within a body cavity according to claim 1, wherein at least one of the end elements (2A, 2B) is moveable along a predetermined part of the tubular body (1) and wherein the moveable end element (2A, 2B) further comprises a locking element (8) for locking the moveable end element (2A, 2B) at a position, wherein the device has expanded the volume of the body cavity.

3. A device for transmitting light within a body cavity according to any of claims 1 or 2, wherein the device has increased the volume of the body cavity at least by 3%, such as at least 5%, such as at least 10%, such as at least 20%.

4. A device for transmitting light within a body cavity according to any of the preceding claims, further comprising a groove and/or a through-going opening (11) for providing air from the surroundings outside the body cavity to the void (10).

5. A device for transmitting light within a body cavity according to any of the preceding claims, further comprising a sensor to be electrically connected to the battery and configured to measure pH and/or temperature and/or colour of a tissue inside the body cavity and/or colour of a fluid inside the body cavity, and wherein the sensor is arranged in the penetration cap (5) and/or in the outer part (3).

6. A device for transmitting light within a body cavity according to any of the preceding claims, wherein the device further comprises an extending element (7), which extends outside the body cavity when the device is inserted within the body cavity.

7. A device for transmitting light within a body cavity according to claim 6 when dependant on claim 5, wherein the extending element (7) is configured to facilitate signal transmission from the sensor to a receiving device positioned outside the body cavity during use of the device.

8. A device for transmitting light within a body cavity according to any of the preceding claims, further comprising an exterior part, wherein the exterior part at least partly covers an outer surface of the body cavity, being the outer surface, which at least forms the entrance to the body cavity.

9. A device for transmitting light within a body cavity according to claim 8, wherein the exterior part comprises a light source configured to provide light at a predetermined wavelength to the surroundings of the exterior part and electrically connected to the switch.

10. A device for transmitting light within a body cavity according to any of the preceding claims, wherein the light provided by the light source is at a wavelength of at least 250 nm, such as at least 300 nm, such as at least 350 nm, such as at least 400 nm, but not at a wavelength above 500 nm.

11. A device for transmitting light within a body cavity according to any of the preceding claims, wherein the outer part (3) is a balloon structure and configured to be inflated such as to expand the body cavity.

12. A device for transmitting light within a body cavity according to any of the preceding claims, wherein the length of the device is between 3 and 15 cm, such as 5 and 11 cm, and the circumference of the device in a not expanded configuration is between 4 and 12 cm, such as 6 and 10 cm.

13. A device for transmitting light within a body cavity according to any of the preceding claims, further comprising a plurality of light sources, such as a plurality of LEDs, such as a plurality of light bulbs, and wherein each light source has a preselected wavelength.

14. A method for transmitting light within a body cavity, the method comprising:
- providing a device for transmitting light, the device comprising
- a main tubular body having a distal end and a proximal end,
- at least one light source positioned within the main tubular body, configured to provide light at a preselected wavelength and arranged to emit said light to the surroundings of the device,
- a switch,
wherein the device is configured to receive a battery to be electrically connected to the switch to power the light source, and wherein the device is adapted to be inserted within the body cavity, such as a vagina,
- inserting said device within a body cavity,
- activating using a switch at least one light source positioned within a main tubular body of the device, the at least one light source being configured to provide light at a preselected wavelength, and wherein said light source is arranged to emit said light to the surroundings of the device,
- removing said device from said body cavity.

15. A method for transmitting light within a body cavity according to claim 14, wherein, upon inserting said device within a body cavity, expanding the body cavity by providing a bending of an outer part of the device, and preferably locking the bended outer part by a locking element, such as a cam locking mechanism.
